Europäisches Patentamt

European Patent Office

Office européen des brevets

(11) Veröffentlichungsnummer : **0 017 972 B1**

(12) **EUROPÄISCHE PATENTSCHRIFT**

(45) Veröffentlichungstag der Patentschrift :
09.06.82

(51) Int. Cl.³ : **C 07 C119/048, B 01 J 8/24**

(21) Anmeldenummer : **80102037.1**

(22) Anmeldetag : **16.04.80**

(54) **Gewinnung von Toluylendiisocyanat und/oder höhersiedenden Lösungsmitteln im Wirbelbett aus Destillationsrückständen der Toluylendiisocyanat-Herstellung.**

(30) Priorität : **19.04.79 DE 2915830**

(43) Veröffentlichungstag der Anmeldung :
**29.10.80 (Patentblatt 80/22)**

(45) Bekanntmachung des Hinweises auf die Patenterteilung : **09.06.82 Patentblatt 82/23**

(84) Benannte Vertragsstaaten :
**BE DE FR GB NL**

(56) Entgegenhaltungen :
EP - A - 0 000 463
DE - A - 2 452 805
FR - A - 1 378 442
FR - A - 2 320 938
US - A - 3 457 291
US - A - 3 897 314

CHEMICAL ABSTRACTS, Band 81, Nr. 20, 18. November 1974, Seite 197, Ref. 123669e A.S. VLASOV et al. : « Extraction of elemental sulfur from volcanicsulfur ores in a fluidized bed »

(73) Patentinhaber : **BASF Aktiengesellschaft**
**Carl-Bosch-Strasse 38**
**D-6700 Ludwigshafen (DE)**

(72) Erfinder : **Koehler, Waldemar, Dr.**
**Max-Slevogt-Strasse 28**
**D-6710 Frankenthal (DE)**
Erfinder : **Blumenberg, Bernd, Dr. c/o BWC**
**P.O.Box 457**
**Geismar, Louisiana 70734 (US)**
Erfinder : **Vogel, Ludwig**
**Carostrasse 8**
**D-6710 Frankenthal (DE)**
Erfinder : **Hetzel, Eckhard**
**Sandweg 26**
**D-6712 Bobenheim-Roxheim (DE)**

Imprimerie Jouve, 18, rue St-Denis, 75001 Paris, France

**0 017 972**

Gewinnung von Toluylendiisocyanat und/oder höhersiedenden Lösungsmitteln im Wirbelbett aus Destillationsrückständen der Toluylendiisocyanat-Herstellung

Die Erfindung betrifft die Abtrennung von Toluylendiisocyanat und/oder höhersiedenden Lösungsmitteln aus Destillationsrückständen, die bei der Herstellung von Toluylendiisocyanat durch Phosgenierung von Toluylendiamin entstehen, durch Eindampfen in einer Wirbelschicht bei Temperaturen von 140 bis 280 °C.

Es ist z.B. aus Ullmanns Encyklopädie der technischen Chemie, 4. Auflage (1977), Bande 13, S. 351, bekannt, daß die Umsetzung von Toluylendiamin mit Phosgen zu Toluylendiisocyanat in Gegenwart eines tiefer als das Toluylendiisocyanat siedenden organischen Lösungsmittels wie etwa Monochlorbenzol oder o-Dichlorbenzol vorgenommen wird. Von verschiedenen Seiten ist auch die Verwendung von höher als Toluylendiisocyanat siedenden Lösungsmitteln vorgeschlagen worden, z.B. Methyldiphenylmethan, Tetrahydronaphthalin oder Alkyl(iso)phthalate (letzteres : FR 1 476 755). Im allgemeinen wird die Phosgenierung in zwei Stufen durchgeführt, zuerst bei Temperaturen von 0 bis 100 °C, anschließend bei 150 bis 180 °C. Dann wird in der Regel die Reaktionslösung durch Abdestillieren oder durch Durchleiten von Stickstoff von restlichem Phosgen befreit und anschließend in einer destillativen Aufarbeitung in das Toluylendiisocyanat, das Lösungsmittel und Nebenprodukte der Phosgenierung, das sind überwiegend nicht flüchtige Rückstände, aufgetrennt.

Sofern für die Phosgenierung ein tiefer als Toluylendiisocyanat siedendes Lösungsmittel eingesetzt wird, wird während des Aufarbeitungsganges zweckmäßigerweise zuerst das Lösungsmittel abdestilliert. Zurück bleibt ein Roh-Toluylendiisocyanat-Gemisch, das in der Regel 65 bis 98 Gew.% Toluylendiisocyanat, 0 bis 10 Gew.% tiefersiedendes Lösungsmittel und 2 bis 25 Gew.% nicht flüchtige Rückstände enthält. Wird dagegen ein höher als Toluylendiisocyanat siedendes Lösungsmittel verwendet, so wird zuerst Toluylendiisocyanat allein oder gemeinsam mit einem Teil des höhersiedenden Lösungsmittel abdestilliert. Zurück bleibt eine Rückstandsmischung, die 65 bis 98 Gew.% des höhersiedenden Lösungsmittels, 0 bis 10 Gew.% restliches Toluylendiisocyanat und 2 bis 25 Gew.% nicht flüchtige Rückstände enthält. Die Rückstände können hierbei ganz oder teilweise gelöst sowie auch teilweise suspendiert vorliegen.

Die vorgenannten Gemische werden nun durch Destillation weiter eingeengt, im ersten Fall zur Gewinnung des Toluylendiisocyanats, im zweiten Fall zur Rückgewinnung des höhersiedenden Lösungsmittels. Hierbei fällt ein Destillationsrückstand an, der noch beträchtliche Mengen, üblicherweise zwischen 20 und 80 Gew.%, an Toluylendiisocyanat und/oder höhersiedendem Lösungsmittel — beides im folgenden als Wertstoff bzw. Wertstoffe benannt — enthält. Die restlichen Bestandteile des Destillationsrückstandes bestehen je nach Herstellungsbedingungen im wesentlichen aus 1 bis 80 Gew.% Harnstoffverbindungen, 0 bis 40 Gew.% Uretdionen, 0 bis 60 Gew.% Isocyanursäureestern, 0,5 bis 20 Gew.% Carbodiimiden und 5 bis 95 Gew.% höher kondensierten oder polymeren Stoffen.

Die Abtrennung der Wertstoffe, also des Toluylendiisocyanats und/oder des höhersiedenden Lösungsmittels, ist für die Wirtschaftlichkeit des Herstellungsverfahrens von maßgebender Bedeutung. Für die Rückgewinnung von Isocyanat aus Rückstandsgemischen wurden verschiedene Verfahren entwickelt. Beschrieben wird beispielsweise in der DE-AS-12 43 178 ein Extraktionsverfahren, in der GB-PS-1 117 066 und DE-AS-12 31 689 Destillationsverfahren und in der DE-AS-19 62 598 ein Hydrolyseverfahren mit anschließender Phosgenierung der Hydrolyseprodukte. Nachteilig an den genannten Verfahren ist, daß zur Isolierung des Isocyanats stets größere Mengen an Lösungs- oder Verdünnungsmitteln benötigt werden, die ihrerseits in separaten kostspieligen Verfahrensschritten gereinigt werden müssen.

Zur Vermeidung dieser Nachteile wurde z.B. vorgeschlagen, den Destillationsrückstand in Schneckenmaschinen zu behandeln oder in ein erwärmtes, mit einem Wendelrührer ausgestattetes Schüttbett so einzubringen, daß er beim Rühren in der vertikalen Mittelzone mit dem Feststoffstrom nach unten geführt wird, dort nach und nach die flüchtigen Bestandteile verliert und in Form eines trockenen, festen Rückstands vom Boden an den peripheren Zonen nach oben bewegt wird (DE-OS 24 52 805). Das letztere Verfahren ist sowohl für die Abtrennung von Toluylendiisocyanat als auch von höhersiedendem Lösungsmittel geeignet. Obwohl diese Verfahren recht günstige Ergebnisse zeigen, weisen sie gewisse Nachteile auf. Nachteilig ist beispielsweise, daß die Apparate bewegliche Teile besitzen, die gewartet werden müssen, wobei die chlor- und chloridhaltigen Destillationsrückstände zusätzlich stark korrodierend wirken.

Ganz allgemein bekannt war ferner, daß Schüttgut mit Hilfe von Wirbelschichten getrocknet und gekühlt werden kann (Maschinenmarkt, Würzburg, 81 (1975) 79, Seiten 1 480 bis 1 973).

Aufgabe der vorliegenden Erfindung war es ein Verfahren zur Abtrennung von Toluylendiisocyanat und/oder höhersiedenden Lösungsmitteln aus Destillationsrückständen, wie sie üblicherweise bei der Toluylendiisocyanatherstellung gebildet werden, zu entwickeln, das ohne zusätzliche Mitverwendung von Extraktions-, Lösungs- oder Destillationshilfsmitteln zufriedenstellende Ergebnisse liefert.

Diese Aufgabe konnte gelöst werden durch ein Verfahren zur Abtrennung von Toluylendiisocyanat und/oder höhersiedenden Lösungsmitteln aus Destillationsrückständen, die bei der Umsetzung von Toluylendiamin mit Phosgen in Gegenwart von Lösungsmitteln und anschließender Destillation der Reaktionslösung erhalten werden, das dadurch gekennzeichnet ist, daß man die Destillationsrückstände

2

in einer Wirbelschicht bei Temperaturen von 140 °C bis 280 °C behandelt.

Überraschend war hierbei insbesondere, daß die hochviskosen bis festen Destillationsrückstände, die in ganz oder teilweise geschmolzenem Zustand in die Wirbelschicht eingedüst werden, nicht die Öffnungen im Anströmboden des Wirbelreaktors verstopfen oder zu größeren, unter wirtschaftlichen Bedingungen nicht mehr wirbelfähigen Agglomeraten zusammenbacken, sondern ein wirbelfähiges Gut bilden, das sich kontinuierlich aus dem Wirbelbett austragen läßt. Trotz der breiten Verweilzeitverteilung im Wirbelbett werden die abzutrennenden Wertstoffe — beim Toluylendiisocyanat handelt es sich um übliche Isomerengemische aus 2,4- und 2,6-Toluylendiisocyanat — thermisch nicht geschädigt, dementsprechend ist auch keine zusätzliche Rückstandsbildung festzustellen. Die in den Destillationsrückständen enthaltenen Wertstoffe werden mit hoher Ausbeute zurückgewonnen.

Vorteilhaft ist ferner, daß die Vorrichtung keine mechanisch bewegten Teile besitzt, wie sie in Schneckenmaschinen oder in gerührten Festbetten vorhanden sind. Daraus ergeben sich eine geringe Reparaturanfälligkeit, geringe Wartungs- und Investitionskosten und hohe Raum-Zeit-Ausbeuten. Da die Abtrennung der Wertstoffe bei Normaldruck erfolgt, treten keine Dichtungsprobleme auf. Die erforderliche Wärme kann einfach über das Wirbelgas zugeführt und an das Wirbelbett übertragen werden ; Hilfslösungsmittel sind nicht erforderlich.

Schließlich ist ein weiterer Vorteil des erfindungsgemäßen Verfahrens, daß der wertstofffreie Trockenrückstand in einer leicht handhabbaren, rieselfähigen und staubarmen Form anfällt.

Zur Durchführung des erfindungsgemäßen Verfahrens eignen sich übliche Wirbelbettanlagen. Vorzugsweise verwendet werden jedoch Sprühwirbelbettanlagen (Abbildung), die im wesentlichen bestehen aus einem vorzugsweise zylinderförmigen Wirbelbehälter (1), dessen Innendurchmesser zur Länge im allgemeinen 1 : 0,5 bis 1 : 7, vorzugsweise 1 : 1 bis 1 : 3 beträgt, mit dem Wirbelbett (2), dem Anströmboden (3) für das Wirbelgas, der beispielsweise aus einer Frittenplatte, einem Rundloch- oder Glockenboden oder einem Conidur-Feinlochblech bestehen kann, einer Düse (4) für die Produktzufuhr, dem Produktabzug (5), der beispielsweise mittels einer Förderschnecke durchgeführt werden kann, und der Abgasabfuhr (6), die gegebenenfalls über ein im Wirbelbehälter befindliches Filter (7) erfolgen kann, sowie gegebenenfalls einer mechanischen Zerkleinerungsvorrichtung (8) mit eventuell vorgeschalteter Klassiereinrichtung (18), beispielsweise einer Mühle oder einem rotierenden Schlagkreuz, die innerhalb oder außerhalb des Wirbelbehälters angeordnet sein kann und einer eventuell auftretenten Kornvergrößerung entgegenwirkt sowie neue Granulationskeime schafft, und Heizflächeneinbauten (9).

Das erfindungsgemäße Verfahren läuft im Prinzip nach folgendem Verfahrensschema ab : Auf getrockneten, bereits im wesentlichen wertstofffreien Rückstand, der als Vorlage der Wirbelschicht (2) dient und eine bestimmte Korngrößenverteilung aufweist, wird bei erhöhten Temperaturen der erhitzte, ganz oder teilweise aufgeschmolzene Destillationsrückstand, der über die Zuleitung (10) geführt und mit einem unter den Reaktionsbedingungen inerten Zerstäubungsgas, das über die Zuleitung (11) geführt wird, in der Zweistoffdüse (4) vereinigt wird, aufgesprüht. Dabei werden die einzelnen Partikel der Wirbelschicht von einer dünnen Schicht des Destillationsrückstandes umgeben, aus der die rückzugewinnenden Wertstoffe (bzw. nur einer) rasch abdestillieren. Durch das Wirbelgas, das über die Zuleitung (12) und den Anströmboden (3) eingebracht wird, wird der Wirbelschicht die zur Verdampfung der Wertstoffe erforderliche Energiemenge teilweise oder ganz zugeführt. Die Mischung aus dampfförmigen Wertstoffen und Wirbelgas wird über die Ableitung (6) dem Filter (7) zugeführt und dort entstaubt ; anschließend werden die Wertstoffe im Kondensator (13) verflüssigt und vom Wirbelgas abgetrennt. Das Wirbelgas wird über die Ableitung (14), ein Gebläse (19) und einen Erhitzer (21) in die Zuleitung (12) eingespeist und so in den Kreislauf zurückgeführt. Ein infolge der Zugabe von Zerstäubungsgas auftretender Gasüberschuß im Wirbelgaskreislauf kann gegebenenfalls über die Leitung (20) abgeführt werden. Vorteilhafterweise wird ein Gasüberschuß dadurch vermieden, daß ein Teil des rückzuführenden Wirbelgases abgezweigt, mittels eines Verdichters (22) komprimiert, über einen Wärmeaustauscher (23) aufgeheizt und über die Leitung (11) als Zerstäubungsgas eingesetzt wird. Die Wertstoffe (bzw. nur einer) fließen über die Ableitung (15) in einen Lagerbehälter. Der trockene, wertstofffreie Rückstand wird kontinuierlich mit einer Förderschnecke über den Produktabzug (5) aus dem Wirbelbett ausgetragen, über die Ableitung (16) mit dem Staub aus dem Filter (7) vereinigt und abgeführt (17).

Zu den erforderlichen Reaktionsbedingungen zur Durchführung des erfindungsgemäßen Verfahrens möchten wir im einzelnen noch das Folgende ausführen :

Wie bereits dargelegt wurde, muß die Vorlage der Wirbelschicht, die aus im wesentlichen wertstofffreiem Rückstand besteht, bestimmte Korngrößen besitzen. Korngrößen von 0,5 bis 5 000 μm, vorzugsweise von 100 bis 2 000 μm haben sich besonders bewährt und werden daher bevorzugt verwendet. Sind die Korngrößen zu klein, so wird das Wirbelgut pneumatisch ausgetragen, während sich zu große Teilchen am Anströmboden absetzen und diesen verstopfen. Die Korngröße kann beispielsweise über den Abrieb im Wirbelbett, der abhängig ist von der Fluidisationszahl, d.h. der auf die Lockerungsgeschwindigkeit WL bezogenen Leerraumgeschwindigkeit W, und der Wirbelschichthöhe (mit wachsender Fluidisationszahl und Schichthöhe steigt die Abriebgeschwindigkeit an) oder durch mechanische Zerkleinerungsvorrichtungen im oder außerhalb des Wirbelbettes gesteuert werden.

Der Destillationsrückstand wird aufgeheizt und bei Temperaturen von 50 bis 300 °C, vorzugsweise von 100 bis 230 °C über eine Einbringvorrichtung, beispielsweise Düsen, vorzugsweise eine oder mehrere Einstoff- oder Zweistoffdüsen, in die Wirbelschicht eingesprüht. Die Düsen können so angeordnet sein, daß sie von oben, von der Seite, von unten oder direkt im Wirbelbett sprühen. Aus verfahrenstechnischen und konstruktiven Gründen hat sich die seitliche Anordnung als vorteilhaft erwiesen, wobei die Düsen oberhalb des Anströmbodens so angeordnet sein müssen, daß ein Auftreffen des versprühten Destillationsrückstandes auf den Boden und damit ein Verkleben desselben vermieden wird. Die Flüssigkeitsbelastung der Düse mit Destillationsrückstand ist abhängig von der Größe der Wirbelschicht und beträgt ungefähr 1 bis 500 kg/h pro m$^2$ Anströmboden, vorzugsweise 50 bis 500 kg/h pro m$^2$ Anströmboden. Als Zerstäubungsgas können unter den Reaktionsbedingungen inerte Gase verwendet werden. Vorzugsweise verwendet wird Stickstoff in Mengen von 0,1 bis 5 kg, vorzugsweise von 0,5 bis 1,5 kg je kg Destillationsrückstand, wobei der Druck des Zerstäubungsgases 1,5 bis 10 bar, vorzugsweise 2 bis 4 bar, beträgt.

Für die Zerstäubung geeignete Gase können auch als Wirbelgas verwendet werden. Vorzugsweise verwendet wird Stickstoff, der vorerhitzt auf eine Temperatur von 100 bis 350 °C, vorzugsweise von 150 bis 280 °C über den Anströmboden zugeführt wird. Die Anströmungsgeschwindigkeit für das Wirbelgas unter Normalbedingungen (20 °C und 760 mmHg) beträgt 0,1 bis 2 m/sec, vorzugsweise 0,2 bis 0,8 m/sec. Aus diesen Angaben lassen sich die Anströmungsgeschwindigkeiten unter Betriebsbedingungen leicht errechnen. Aus der Anströmungsgeschwindigkeit des Wirbelgases und dem Korndurchmesser der Vorlage der Wirbelschicht können ferner die Lückengrade $\varepsilon_L$ am Lockerungspunkt berechnet werden. Sie besitzen Werte von 0,4 bis 0,75, vorzugsweise von 0,5 bis 0,7.

Die Abtrennung der Wertstoffe erfolgt in der Wirbelschicht praktisch unter Atmosphärendruck bei Temperaturen von 140 bis 280 °C, vorzugsweise 160 bis 250 °C. Die erforderliche Energie wird vorzugsweise über das Wirbelgas zugeführt und an die Vorlage übertragen. Gegebenenfalls kann es jedoch zweckmäßig sein Heizflächen, beispielsweise Heizplatten oder Rohrbündel, in die Wirbelschicht einzubauen.

Die Verweilzeit des im wesentlichen wertstofffreien Rückstandes in der Wirbelschicht beträgt ungefähr 0,1 bis 10 Stunden, vorzugsweise 0,1 bis 3 Stunden. Nach dem Austrag besitzt der Rückstand einen Restgehalt an Wertstoff von 0,1 bis 2,0 Gewichtsprozent, vorzugsweise von 0,3 bis 1 Gewichtsprozent.

Der Rückstand wird über einen Produktabzug aus der Wirbelschicht ausgetragen. Wesentlich hierbei ist, daß sich nicht mehr wirbelnde Rückstandspartikel nicht am Anströmboden anreichern und den Wirbelprozeß zum Erliegen bringen, sondern daß durch Neigung der Anströmböden zum Produktabzug hin, durch entsprechende Einbauten oder durch Verwendung von Anströmboden mit zum Produktabzug hin gerichteten Wirbelgasstrahlen dafür gesorgt wird, daß die am Boden liegenden Rückstandspartikel zum Produktabzug geschoben und an einer Rückwandung gehindert werden. Der Produktabzug kann beispielsweise über einen Bodenauslaß oder Austragsschacht erfolgen, wobei die Austragsmenge z.B. mit Hilfe eines Abdeckbleches, eines Schiebers, einer Zellenradschleuse oder vorzugsweise von Förderschnecken gesteuert werden kann.

Der ausgetragene Rückstand wird mit dem Rückstand aus der Entstaubungsfilter vereinigt und z.B. verbrannt.

Die abgeführte Mischung aus dampfförmigem Wertstoff oder Wertstoffgemisch und Wirbelgas, die einen Wertstoffgehalt von 0,001 bis 0,3, vorzugsweise von 0,1 bis 1 kg Wertstoff pro kg Wirbelgas besitzt, wird, wie bereits ausgeführt wurde, entstaubt und danach in einem Kondensator auf Temperaturen von 25 bis 160 °C abgekühlt. Hierbei verflüssigt sich der Wertstoff bzw. das Wertstoffgemisch und wird auf diese Weise vom Wirbelgas abgetrennt und danach einem Lagerbehälter zugeleitet.

Das Wirbelgas wird hingegen nach Aufheizung mittels des Wärmetauschers (21) in den Wirbelgaskreislauf zurückgeführt. Ein infolge der Zugabe von Zerstäubungsgas auftretender Gasüberschuß im Wirbelgaskreislauf muß über die Leitung (20) abgeführt werden. Zweckmäßiger ist es, einen Gasüberschuß dadurch zu vermeiden, daß ein Teil des rückzuführenden Wirbelgases abgezweigt, mittels eines Verdichters (22) komprimiert, über einen Wärmetauscher (23) aufgeheizt und über Leitung (11) als Zerstäubungsgas eingesetzt wird.

Die folgenden Beispiele sollen die Erfindung erläutern, ohne sie zu beschränken.

## Beispiele

Allgemeine Verfahrensvorschrift :

In einen von außen elektrisch beheizten, zylinderförmigen Wirbelbehälter mit einem Durchmesser von 6 cm und einer Höhe von 30 cm, ausgestattet mit einer Fritte als Anströmboden und einer Zweistoffdüse, die seitlich 5 cm über dem Anströmboden angeordnet ist, sowie einer Förderschnecke als Produktabzug, werden 80 g trockener Rückstand mit einem durchschnittlichen Korndurchmesser von 0,2 bis 1 mm als Vorlage eingebracht. Das in einem Wärmetauscher erhitzte Wirbelgas Stickstoff wird über den Anströmungsboden zugeführt, der Destillationsrückstand, der einen durchschnittlichen Wertstoffgehalt von 50 bis 75 Gewichtsprozent besitzt, in Form einer Schmelze mit Stickstoff als Zerstäubungsgas eingedüst.

Das Gemisch aus dampfförmigem Wertstoff und Stickstoff wird mit Hilfe eines Drahtgewebefilters entstaubt und in einem Kondensator abgekühlt und getrennt.

Für die nachfolgende aufgeführten Versuche wurden zwei verschiedene Destillationsrückstände verwendet. Zum einen wurde ein Destillationsrückstand (I) eingesetzt, der aus einer in Monochlorbenzol als Lösungsmittel durchgeführten Phosgenierung von Toluylendiamin zu Toluylendiisocyanat stammte und als rückzugewinnenden Wertstoff 68,5 Gewichtsprozent Toluylendiisocyanat (TDI) enthielt. Für die Beispiele zur Rückgewinnung eines höher als Toluylendiisocyanat siedenden Lösungsmittels wurde ein Destillationsrückstand (II) gewählt, der aus einer in Diäthylisophthalat als Lösungsmittel durchgeführten Phosgenierung von Toluylendiamin zu Toluylendiisocyanat gewonnen wurde und 57,0 Gewichtsprozent Diäthylisophthalat (DEIP) als rückzugewinnenden Wertstoff enthielt.

Für die aufgeführten 7 beispielhaften Versuche sind die Versuchsbedingungen, zusätzliche charakteristische Daten und die Resultate in der nachfolgenden Tabelle angegeben. Versuchsdaten, die für alle 7 Beispiele gleich waren und bereits in der obigen allgemeinen Verfahrensvorschrift angegeben sind, sind in der Tabelle nicht mehr enthalten.

(Siehe die Tabelle, S. 6)

Tabelle : Versuchsdaten für die Beispiele 1 bis 7

| | | Beispiele: 1 | 2 | 3 | 4 | 5 | 6 | 7 |
|---|---|---|---|---|---|---|---|---|
| **Wirbelschicht** | | | | | | | | |
| Temperatur | $^{o}C$ | 240 | 170 | 150 | 250 | 250 | 270 | 280 |
| **Destillationsrückstand** | Art | I | I | I | I | II | II | II |
| Zufuhrmenge über Leitung (10) | kg/h | 0,535 | 0,247 | 0,263 | 1,324 | 0,438 | 0,827 | 1,269 |
| Temperatur | $^{o}C$ | 190 | 150 | 150 | 200 | 220 | 220 | 220 |
| **Zerstäubungsgas** | | | | | | | | |
| Zufuhrmenge über Leitung (11) | kg/h | 0,50 | 0,27 | 0,16 | 0,73 | 0,42 | 0,38 | 1,0 |
| Temperatur | $^{o}C$ | 190 | 150 | 150 | 200 | 220 | 220 | 220 |
| Vordruck | bar | 3 | 2 | 1,5 | 5,5 | 2,5 | 2,5 | 9 |
| **Wirbelgas** | | | | | | | | |
| Zufuhrmenge über Leitung (12) | kg/h | 1,0 | 1,0 | 1,3 | 2,0 | 1,0 | 1,5 | 12,5 |
| Temperatur | $^{o}C$ | 260 | 185 | 165 | 280 | 270 | 295 | 290 |
| Wirbelgasgeschwindigkeit, berechnet für Normalbedingungen | m/sec | 0,12 | 0,12 | 0,16 | 0,24 | 0,12 | 0,18 | 1,5 |
| **Wirbelgut** | | | | | | | | |
| Lückengrad $\varepsilon_1$ am Lockerungspunkt | | 0,6 | 0,7 | 0,75 | 0,55 | 0,6 | 0,5 | 0,45 |
| Durchschnittliche Verweilzeit des Rückstands in der Wirbelschicht | min | 27 | 66 | 55 | 12 | 27 | 12 | 9 |

0 017 972

Tabelle (Fortsetzung)

| Beispiele: | | 1 | 2 | 3 | 4 | 5 | 6 | 7 |
|---|---|---|---|---|---|---|---|---|
| Wertstoffaustrag | Art | TDI | TDI | TDI | TDI | DEIP | DEIP | DEIP |
| Wertstoffgehalt des über Leitung (6) abgeführten Gasgemisches | Gew.% | 19,5 | 11,7 | 11,0 | 24,9 | 14,9 | 20,0 | 5,1 |
| Temperatur des Kondensers (13) | °C | 80 | 80 | 70 | 80 | 90 | 90 | 90 |
| Kondensatanfall über Leitung (15) | kg/h | 0,348 | 0,159 | 0,170 | 0,868 | 0,239 | 0,448 | 0,664 |
| Rückgewinnungs--Ausbeute | % | 95,0 | 94,0 | 94,3 | 95,7 | 95,9 | 95,1 | 91,8 |
| Rückstandsaustrag | | | | | | | | |
| Über Leitung (17) abgeführte Rückstandsmenge | kg/h | 0,177 | 0,073 | 0,087 | 0,413 | 0,181 | 0,402 | 0,551 |
| Wertstoff-Restgehalt | Gew.% | 0,7 | 1,6 | 1,8 | 0,5 | 0,85 | 1,2 | 0,3 |
| Korngrößenbereich | mm Ø | 0,1-1,2 | 0,1-3,0 | 0,1-3,5 | 0,05-1,1 | 0,1-1,3 | 0,05-1,2 | 0,05-1,1 |

0 017 972

**0 017 972**

Die Bilanzierung der Versuche bezüglich des Wertstoffs ergab hohe Rückgewinnungs-Ausbeuten zwischen ca. 92 und 96 %. Mit dem trockenen Rückstand wurden nur geringe Mengen an Wertstoff ausgetragen. Die hierdurch auftretenden Wertstoffverluste betrugen 0,2 % (Beispiel 4) bis 0,9 % (Beispiel 3) beim Toluylendiisocyanat und 0,2 % (Beispiel 7) bis 1,0 % (Beispiel 6) beim Diäthylisophthalat. Der größte Teil der Ausbeutedifferenz zu 100 % ist durch den Restgehalt im Abgas (14) nach dem Kondenser (13) zurückzuführen. Die Rückgewinnungsausbeute läßt sich somit bei Kreisführung des Wirbel- und Zerstäubungsgases weiter steigern.

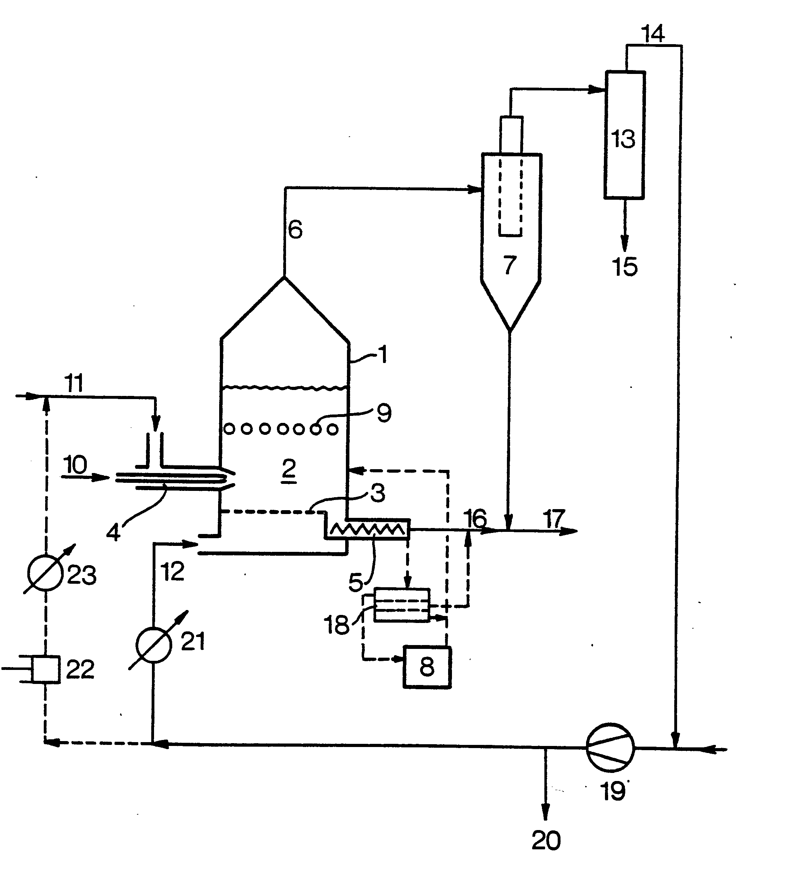

**Ansprüche**

1. Verfahren zur Abtrennung von Toluylendiisocyanat und/oder höhersiedenden Lösungsmitteln aus Destillationsrückständen, die bei der Umsetzung von Toluylendiamin mit Phosgen in Gegenwart von Lösungsmitteln und anschließender Destillation der Reaktionslösung erhalten werden, dadurch gekennzeichnet, daß man die Destillationsrückstände in einer Wirbelschicht bei Temperaturen von 140 °C bis 280 °C behandelt.

2. Verfahren nach Anspruch 1, dadurch gekennzeichnet, daß die Lückengrade $\varepsilon_L$ am Lockerungspunkt 0,4 bis 0,75 sind.

3. Verfahren nach Anspruch 1, dadurch gekennzeichnet, daß die im Wirbelbehälter anfangs vorgelegte Feststoffschüttung eine durchschnittliche Korngröße von 0,5 bis 5 000 µm besitzt.

4. Verfahren nach Anspruch 1, dadurch gekennzeichnet, daß die Anströmungsgeschwindigkeiten für das Wirbelgas unter Normalbedingungen (20 °C und 760 mm Hg) 0,1 bis 2 m/sec. betragen.

5. Verfahren nach Anspruch 1, dadurch gekennzeichnet, daß als Wirbelgas Stickstoff verwendet wird, der im Kreislauf geführt wird.

6. Verfahren nach Anspruch 1, dadurch gekennzeichnet, daß für die Einsprühung des Destillationsrückstands in die Wirbelschicht eine Zweistoffdüse verwendet und das Zerstäubungsgas mit einem Vordruck von 1,5 bis 10 bar in die Wirbelschicht eingedüst wird.

7. Verfahren nach Anspruch 1, dadurch gekennzeichnet, daß die Energie für die Verdampfung des Wertstoffs oder Wertstoffgemisches in der Wirbelschicht über die Vorheizung des Wirbelgases eingebracht wird.

8. Verfahren nach Anspruch 1, dadurch gekennzeichnet, daß die Energie für die Verdampfung des Wertstoffs oder Wertstoffgemischs in der Wirbelschicht über die Wand des Wirbelbehälters und/oder durch in die Wirbelschicht eingebaute Wärmetauscherflächen eingebracht wird.

**Claims**

1. A process for the separation of toluene diisocyanates and/or higher boiling solvents from distillation residues obtained in the reaction of toluene diamine with phosgene in the presence of solvents, and subsequent distillation of the reaction solution, characterized in that the distillation residues are treated in a fluidized bed at temperatures of from 140 °C to 280 °C.

2. A process as claimed in claim 1, characterized in that the void fractions $\varepsilon_L$ at the fluidizing point are from 0.4 to 0.75.

3. A process as claimed in claim 1, characterized in that the solid material initially placed in the fluidized bed vessel has an average particle size of from 0.5 to 5 000 µm.

4. A process as claimed in claim 1, characterized in that the initial flow velocity of the fluidizing gas is from 0.1 meter per second to 2 meters per second under standard conditions (20 °C and 760 mmHg).

5. A process as claimed in claim 1, characterized in that nitrogen, which is recycled, is used as the fluidizing gas.

6. A process as claimed in claim 1, characterized in that a two-fluid nozzle is used to inject the distillation residue into the fluidized bed and the atomizing gas is injected into the fluidized bed with a supply pressure of 1.5 bars to 10 bars.

7. A process as claimed in claim 1, characterized in that the energy for evaporating the useful product or mixture of useful products in the fluidized bed is introduced by preheating the fluidizing gas.

8. A process as claimed in claim 1, characterized in that the energy for evaporating the useful product or mixture of useful products in the fluidized bed is introduced bia the wall of the fluidized bed vessel and/or via heat exchange surfaces installed in the fluidized bed.

**Revendications**

1. Procédé pour l'extraction du diisocyanate de toluylène et(ou) de solvants à point d'ébullition plus élevé des résidus de distillation provenant de la réaction de la toluylène-diamine avec le phosgène en présence de solvants, suivie de la distillation de la solution réactionnelle, caractérisé en ce que les résidus de distillation sont traités dans un lit fluidisé à des températures de 140 à 280 °C.

2. Procédé suivant la revendication 1, caractérisé en ce qu'au point de fluidisation, les taux de vides $\varepsilon_L$ se situent entre 0,4 et 0,75.

3. Procédé suivant la revendication 1, caractérisé en ce que la matière solide de remplissage, introduite initialement dans la chambre de fluidisation, possède une granulométrie moyenne de 0,5 à 5 000 μm.

4. Procédé suivant la revendication 1, caractérisé en ce que, dans des conditions normales (20 °C et 760 mm de Hg), les vitesses de soufflage du gaz de fluidisation sont comprises entre 0,1 et 2 m/s.

5. Procédé suivant la revendication 1, caractérisé en ce que le gaz de fluidisation est de l'azote circulant en circuit fermé.

6. Procédé suivant la revendication 1, caractérisé en ce que le résidu de distillation est introduit dans le lit fluidisé à travers une buse de pulvérisation pour deux matières et le gaz de pulvérisation est injecté dans le lit fluidisé sous une pression de 1,5 à 10 bars.

7. Procédé suivant la revendication 1, caractérisé en ce que l'énergie nécessaire pour la vaporisation du produit recherché ou du mélange de produits recherchés dans le lit fluidisé est fournie par un préchauffage du gaz de fluidisation.

8. Procédé suivant la revendication 1, caractérisé en ce que l'énergie nécessaire pour la vaporisation du produit recherché ou du mélange de produits recherchés dans le lit fluidisé est fournie par la paroi de la chambre de fluidisation et (ou) par des surfaces échangeuses de chaleur noyées dans le lit fluidisé.